(19) Europäisches Patentamt / European Patent Office / Office européen des brevets

(11) **EP 3 698 370 B1**

(12) **EUROPEAN PATENT SPECIFICATION**

(45) Date of publication and mention
of the grant of the patent:
**08.04.2026 Bulletin 2026/15**

(21) Application number: **18786788.2**

(22) Date of filing: **19.10.2018**

(51) International Patent Classification (IPC):
**G16H 20/17** (2018.01)    **G16H 40/63** (2018.01)
**A61B 5/00** (2006.01)    *A61B 5/145* (2006.01)

(52) Cooperative Patent Classification (CPC):
**A61B 5/4839; G16H 20/17; G16H 40/63;**
A61B 5/14532

(86) International application number:
**PCT/EP2018/078682**

(87) International publication number:
**WO 2019/077095 (25.04.2019 Gazette 2019/17)**

(54) **BOLUS CALCULATOR AND METHOD FOR CALCULATING A BOLUS**

BOLUSRECHNER UND VERFAHREN ZUR BERECHNUNG EINES BOLUS

CALCULATEUR DE BOLUS ET PROCÉDÉ POUR CALCULER UN BOLUS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.10.2017 EP 17306414**

(43) Date of publication of application:
**26.08.2020 Bulletin 2020/35**

(60) Divisional application:
**26160891.3**

(73) Proprietor: **Sanofi**
**75008 Paris (FR)**

(72) Inventors:
- **KLEMM, Thomas**
  **65926 Frankfurt (DE)**
- **SCHABBACH, Michael**
  **65926 Frankfurt am Main (DE)**

(74) Representative: **Weilnau, Carsten et al**
**Patentanwälte Sturm Weilnau Franke**
**Partnerschaft mbB**
**Unter den Eichen 5 (Haus C-Süd)**
**65195 Wiesbaden (DE)**

(56) References cited:
**US-A- 5 822 715      US-A1- 2009 006 129
US-A1- 2010 295 686      US-A1- 2011 077 493**

**Description**

Technical Field

The disclosure generally relates to a bolus calculator and to a method for calculating a bolus.

Background

WO 2013/184896 A1 discloses that diabetes mellitus, often referred to as diabetes, is a chronic condition in which a person has elevated blood glucose levels that result from defects in the body's ability to produce and/or use insulin. There are three main types of diabetes. Type 1 diabetes usually strikes children and young adults, and may be autoimmune, genetic, and/or environmental. Type 2 diabetes accounts for 90-95% of diabetes cases and is linked to obesity and physical inactivity. Gestational diabetes is a form of glucose intolerance diagnosed during pregnancy and usually resolves spontaneously after delivery.

Without treatment, diabetes can lead to severe complications such as heart disease, stroke, blindness, kidney failure, amputations, and death related to pneumonia and flu.

Management of diabetes is complex as the level of blood glucose entering the bloodstream is dynamic. The variation of insulin that controls the transport of glucose out of the bloodstream also complicates diabetes management. Blood glucose levels are sensitive to diet and exercise, but also can be affected by sleep, stress, smoking, travel, illness, menses, and other psychological and lifestyle factors unique to individual patients. The dynamic nature of blood glucose and insulin, and all other factors affecting blood glucose, often require a person with diabetes to understand ongoing patterns and forecast blood glucose levels (or at least understand the actions that raise or lower glucose in the body). Therefore, therapy in the form of insulin or oral medications, or both, can be timed to maintain blood glucose levels in an appropriate range.

Management of diabetes is often highly intrusive because of the need to consistently obtain reliable diagnostic information, follow prescribed therapy, and manage lifestyle on a daily basis. Daily diagnostic information, such as blood glucose, is typically obtained from a capillary blood sample with a lancing device and is then measured with a handheld blood glucose meter. Interstitial glucose levels may be obtained from a continuous glucose sensor worn on the body.

Prescribed therapies may include insulin, oral medications, or both. Insulin can be delivered with a syringe, an insulin pen, an ambulatory infusion pump, or a combination of such devices. With insulin therapy, calculating the amount of insulin to be injected can require determining meal composition of carbohydrates, fat and proteins along with effects of exercise or other physiologic states. The management of lifestyle factors such as body weight, diet, and exercise can significantly influence the type and effectiveness of a therapy.

Management of diabetes involves large amounts of diagnostic data and prescriptive data that are acquired from medical devices, personal healthcare devices, patient recorded information, healthcare professional biomarker data, prescribed medications and recorded information. Medical devices including self-monitoring bG meters, continuous glucose monitors, ambulatory insulin infusion pumps, diabetes analysis software, and diabetes device configuration software each of which generates and/or manages large amounts of diagnostic and prescriptive data. Personal healthcare devices include weight scales, pedometers and blood pressure cuffs. Patient recorded information includes information relating to meals, exercise and lifestyle as well as prescription and non-prescription medications. Healthcare professional biomarker data includes HbA1C, fasting glucose, cholesterol, triglycerides and glucose tolerance test results. Healthcare professional recorded information includes therapy and other information relating to the patient's treatment.

There is a need for a patient device to aggregate, manipulate, manage, present, and communicate diagnostic data and prescriptive data from medical devices, personal healthcare devices, patient recorded information, biomarker information and recorded information in an efficient manner to improve the care and health of a person with diabetes, so the person with diabetes can lead a full life and reduce the risk of complications from diabetes.

Additionally, there is a need for a diabetes management device that is able to provide an even more accurate bolus recommendation to the user based on various user inputs that take into account recent activities and events which may have an effect on the bG level of a patient to thus enhance the accuracy, convenience and/or efficiency of the device in generating a recommended bolus or a suggested carbohydrate amount for the user.

Prior art publication US2009/006129 A1 discloses such systems and methods for bolus recommendations.

There remains a need for an improved bolus calculator and an improved method for calculating a bolus.

Summary

An object of the present disclosure is to provide an improved bolus calculator and an improved method for calculating a bolus.

The object is achieved by a bolus calculator according to claim 1 and a method according to claim 6.

Exemplary embodiments are provided in the dependent claims.

According to the present disclosure, a bolus calculator for determining a bolus of insulin has an input configured to be fed with a time series of blood glucose values and to store at least one known pulse response representing an active profile of at least one insulin, wherein the bolus calculator is configured to convolute the time series of blood glucose values with the known pulse response to obtain the bolus.

The active profile of a rapid acting insulin shows a short but high insulin level in the blood while the same amount of a slowly acting insulin shows a long and flat active level. The bolus of medicament corresponds with the area under the active level curve, i.e. the integral of the concentration in the blood over time.

Delivery of insulin, e.g. by a pen, causes occurence of a pulse similar to a Dirac pulse of the insulin. This way, an active agent, i.e. the insulin, is suddenly introduced into the system being a human body and the system responds with a step response. When using a pump, delivery can be performed very slowly or in small, repetitive injections (e.g. pulse like, similar to a Dirac pulse).

The active profile is the pharmacokinetic mode of action of different insulins which may act over different time spans and may achieve different levels of insulin in the blood. This can be tested by injecting an insulin and measuring the blood glucose level over time to obtain the pulse response to the respective insulin in the blood glucose level. Likewise, clamping experiments may be performed to show the free insulin level at constant glucose level. The trends can then be normalized.

Typically, each insulin has a specific pharmacokinetic mode of action. Typically, half life periods are used to indicate when the effect of the insulin has decayed to half the initial or maximum level.

In an exemplary embodiment, the bolus calculator is further configured to take into account a dead time in the time series due to blood glucose measurement of capillary blood of a human body.

In an exemplary embodiment, the bolus calculator is further configured to calculate a bolus for one type of insulin.

In an exemplary embodiment, the types of insulin comprise a slowly acting insulin, a rapid-acting insulin and a medium acting insulin.

In an exemplary embodiment, the bolus calculator is further configured to store and take into account patient specifics, in particular at least one of age, sex, weight and body fat percentage. In an exemplary embodiment, the bolus calculator is further configured to store a characteristic map comprising a plurality of stored pulse responses for one or more types of insulin for different sets of patent specifics.

In an exemplary embodiment, the bolus calculator is further configured to convolute all selectable types of insulin for the set patient specifics with the time series of measured blood glucose values and select the most appropriate type of insulin.

In an exemplary embodiment, the bolus calculator is further configured to perform a teach-in run to measure a pulse response of the human body in order to determine the dead time.

In an exemplary embodiment, the bolus calculator is further configured to store measured blood glucose values and boluses calculated to generate a history and configured to take the history into account when calculating boluses.

In an exemplary embodiment, an arrangement for determining a bolus of insulin comprises the bolus calculator, a human body model, a blood glucose sensor adapted to perform a blood glucose measurement of venous blood or capillary blood of a human body and a comparator for comparing blood glucose values measured by the blood glucose sensor with blood glucose values calculated by the human body model before handing over to the bolus calculator.

The body model is a complex controlled system which is subjected to the pulses of the injections and which is permanently impacted by the metabolism, food intake, activity, day/night rhythm, age, sex and body mass or weight and by the type of disease. The insulin sensitivity factor is variable with type 2 diabetes. A health tracker may be arranged to acquire the consumed calories by means of the heart rate (c.f. Harris-Benedict formula which is an established standard method with adaptations for determining the consumed calories) as a function of weight, resting heart rate, age, sex and current heart rate.

In an exemplary embodiment, the blood glucose sensor is adapted to perform the blood glucose measurement continuously, periodically, randomly or pseudo-randomly.

In an exemplary embodiment, the arrangement further comprises a controller, wherein the bolus calculator is arranged in the controller.

In an exemplary embodiment, the bolus calculator is adapted to transmit the bolus to a drug delivery device, which is adapted to automatically set the bolus.

In an exemplary embodiment, the bolus calculator is connected to a drug delivery device and adapted to control delivery of the bolus and optionally the type of insulin selected.

In an exemplary embodiment, the blood glucose sensor is attached to or implanted in a human body and adapted to determine blood glucose values by capillary glucose measurement or by venous glucose measurement.

In an exemplary embodiment, the controller or the arrangement is a mobile device.

In an exemplary embodiment, the controller is configured to communicate with the blood glucose sensor by a wired or wireless connection.

According to an aspect of the present disclosure, a method for calculating a bolus of insulin comprises feeding a time series of blood glucose values into an input of a bolus calculator and storing at least one known pulse response representing an active profile of at least one insulin in the bolus calculator, convoluting the time series of blood glucose values with the known pulse response to obtain the bolus.

In an exemplary embodiment, a dead time in the time series due to blood glucose measurement of capillary blood of a human body is taken into account.

In an exemplary embodiment, a bolus for one type of insulin is calculated or one out of a plurality of types of insulin is selected and the bolus for this selected type is calculated.

In an exemplary embodiment, the types of insulin comprise a slowly acting insulin, a rapid-acting insulin and a medium acting insulin.

In an exemplary embodiment, the method further comprises storing and taking into account patient specifics, in particular at least one of age, sex, weight and body fat percentage.

In an exemplary embodiment, the method further comprises storing a characteristic map comprising a plurality of stored pulse responses for one or more types of insulin for different sets of patient specifics.

In an exemplary embodiment, the method further comprises convoluting all selectable types of insulin for the set patient specifics with the time series of measured blood glucose values and selecting the most appropriate type of insulin.

In an exemplary embodiment, the method further comprises performing a teach-in run to measure a pulse response of the human body in order to determine the dead time.

In a teach-in run, parameters may be adjusted to the patient and the complex body model control loop is loaded with values, inter alia continuous glucose measurement values. This may work best at times in which no insulin is being delivered, e.g. at night. In a subsequent phase, insulin may be delivered as previously normal while the values keep being fed into the body model to keep teaching it for optimizing parameters. The injections may be extrapolated by a continuous simulation and be compared with actual values of the continuous glucose measurement and the hitherto used algorithm to obtain a vernier adjustment of the parameters and indirectly determine the insulin resistence. If the control loop has approximated the actual values and the simulation, teach-in mode may be exited to enter an active mode.

In the active mode, the body model has been taught-in and life parameters are continuously fed in, e.g. continuous glucose measurement values, carbs (food calories, e.g. by manual input), heart rate by a health tracker. Calory intake also cause a kind of pulse response in the system. The control circuit may now try to extrapolate the new values by simulation (including the past trend), e.g. in order to estimate how delivery of one insuline or another one at a certain point in time affects the glucose level course. The objective is to minimize glucose level fluctuations around a defined target value, e.g. 100mg/dL. If an optimum is identified it may be output. As the control circuit knows the available types of insulin it can permutate them in the simulation. This may be achieved by repeated simulation towards the glucose target range. The patient typically decides when to perform the injections (typically after the meals). The optimization of the bolus may then be performed at that point in time, either outputting a value which is to be confirmed or by controlling a pump which then may deliver the bolus.

It is known that the active profiles of all insulins are equal or converge at the end of the active period and cannot be neglected (e.g. Apidra® continues to have an effect even after 24 hours). Dead time may have to be considered as the glucose level is not equal in all parts of the body and everything arrives in each part of the body with a certain delay. In an exemplary embodiment, the method further comprises storing measured blood glucose values and boluses calculated to generate a history and taking the history into account when calculating boluses.

In an exemplary embodiment, the method further comprises comparing blood glucose values measured by a blood glucose sensor with blood glucose values calculated by a human body model before handing over to the bolus calculator.

In an exemplary embodiment, the blood glucose measurement is performed continuously, periodically, randomly or pseudo-randomly.

In an exemplary embodiment, the bolus is transmitted to a drug delivery device, in which the bolus is automatically set.

In an exemplary embodiment, the method further comprises controlling delivery of the bolus and optionally selecting the type of insulin.

The improved bolus calculator and the improved method may more exactly and reliably determine a bolus of insulin to be used in intensified diabetes therapy, in particular with a number of different types of insulin having different active profiles.

A drug delivery device, as described herein, may be configured to inject a medicament into a patient. For example, delivery could be sub-cutaneous, intramuscular, or intravenous. Such a device could be operated by a patient or care-giver, such as a nurse or physician, and can include various types of safety syringe, pen-injector, or auto-injector. The device can include a cartridge-based system that requires piercing a sealed ampule before use. Volumes of medicament delivered with these various devices can range from about 0.5 ml to about 2 ml. Yet another device can include a large volume device ("LVD") or patch pump, configured to adhere to a patient's skin for a period of time (e.g., about 5, 15, 30, 60, or 120 minutes) to deliver a "large" volume of medicament (typically about 2 ml to about 5 ml).

In combination with a specific medicament, the presently described devices may also be customized in order to operate within required specifications. For example, the device may be customized to inject a medicament within a certain time period (e.g., about 3 to about 20 seconds for auto-injectors, and about 10 minutes to about 60 minutes for an LVD). Other specifications can include a low or minimal level of discomfort, or to certain conditions related to human factors, shelf-life, expiry, biocompatibility, environmental considerations, etc. Such variations can arise due to various factors, such as, for example, a drug ranging in viscosity from about 3 cP to about 50 cP. Consequently, a drug delivery device will often include a hollow needle ranging from about 25 to about 31 Gauge in size. Common sizes are 27 and 29 Gauge.

The delivery devices described herein can also include one or more automated functions. For example, one or more of needle insertion, medicament injection, and needle retraction can be automated. Energy for one or more automation steps can be provided by one or more energy sources. Energy sources can include, for example, mechanical, pneumatic, chemical, or electrical energy. For example, mechanical energy sources can include springs, levers, elastomers, or other mechanical mechanisms to store or release energy. One or more energy sources can be combined into a single device. Devices can further include gears, valves, or other mechanisms to convert energy into movement of one or more components of a device.

The one or more automated functions of an auto-injector may be activated via an activation mechanism. Such an activation mechanism can include one or more of a button, a lever, a needle sleeve, or other activation component. Activation may be a one-step or multi-step process. That is, a user may need to activate one or more activation mechanism in order to cause the automated function. For example, a user may depress a needle sleeve against their body in order to cause injection of a medicament. In other devices, a user may be required to depress a button and retract a needle shield in order to cause injection.

In addition, such activation may activate one or more mechanisms. For example, an activation sequence may activate at least two of needle insertion, medicament injection, and needle retraction. Some devices may also require a specific sequence of steps to cause the one or more automated functions to occur. Other devices may operate with sequence independent steps.

Some delivery devices can include one or more functions of a safety syringe, pen-injector, or auto-injector. For example, a delivery device could include a mechanical energy source configured to automatically inject a medicament (as typically found in an auto-injector) and a dose setting mechanism (as typically found in a pen-injector).

Further scope of applicability of the present disclosure will become apparent from the detailed description given hereinafter.

Brief Description of the Drawings

The present disclosure will become more fully understood from the detailed description given below and the accompanying drawings, which are given by way of illustration only, and do not limit the present disclosure, and wherein:

Figure 1    is a schematic view of a drug delivery device,

Figure 2    is a schematic view of a control loop comprising a controller having a bolus calculator,

Figure 3    is a schematic view of an exemplary embodiment of an arrangement for determining a bolus of insulin,

Figure 4    is a schematic view of an exemplary embodiment of an arrangement for determining a bolus of insulin,

Figure 5    is a schematic view of an exemplary embodiment of an arrangement for determining a bolus of insulin, and

Figure 6    is a schematic view of a simplified body model.

Corresponding parts are marked with the same reference symbols in all figures.

Detailed Description

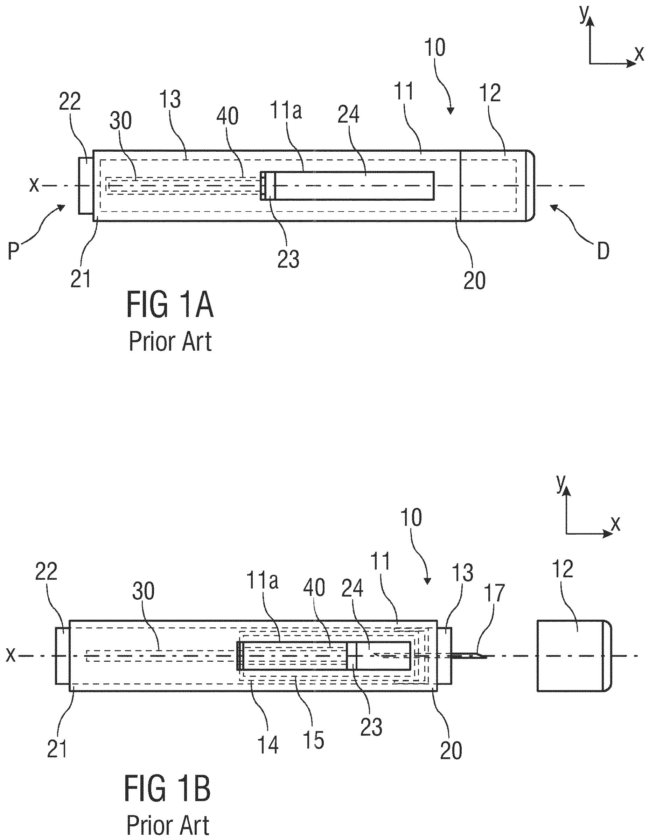

According to some embodiments of the present disclosure, an exemplary drug delivery device 10 is shown in Figs. 1A & 1B. Device 10, as described above, is configured to inject a medicament into a patient's body. Device 10 includes a housing 11 which typically contains a reservoir containing the medicament to be injected (e.g., a syringe) and the components required to facilitate one or more steps of the delivery process. Device 10 can also include a cap assembly 12 that can be detachably mounted to the housing 11. Typically a user must remove cap 12 from housing 11 before device 10 can be operated.

As shown, housing 11 is substantially cylindrical and has a substantially constant diameter along the longitudinal axis X. The housing 11 has a distal region 20 and a proximal region 21. The term "distal" refers to a location that is relatively closer to a site of injection, and the term "proximal" refers to a location that is relatively further away from the injection site.

Device 10 can also include a needle sleeve 13 coupled to housing 11 to permit movement of sleeve 13 relative to housing 11. For example, sleeve 13 can move in a longitudinal direction parallel to longitudinal axis X. Specifically, movement of sleeve 13 in a proximal direction can permit a needle 17 to extend from distal region 20 of housing 11.

Insertion of needle 17 can occur via several mechanisms. For example, needle 17 may be fixedly located relative to housing 11 and initially be located within an extended needle sleeve 13. Proximal movement of sleeve 13 by placing a distal end of sleeve 13 against a patient's body and moving housing 11 in a distal direction will uncover the distal end of needle 17. Such relative movement allows the distal end of needle 17 to extend into the patient's body. Such insertion is termed "manual" insertion as needle 17 is manually inserted via the patient's manual movement of housing 11 relative to sleeve 13.

Another form of insertion is "automated," whereby needle 17 moves relative to housing 11. Such insertion can be triggered by movement of sleeve 13 or by another form of activation, such as, for example, a button 22. As shown in Figs. 1A & 1B, button 22 is located at a proximal end of housing 11. However, in other embodiments, button 22 could be located on a side of housing 11.

Other manual or automated features can include drug injection or needle retraction, or both. Injection is the process by which a bung or piston 23 is moved from a proximal location within a syringe (not shown) to a more distal location within the syringe in order to force a medicament from the syringe through needle 17. In some embodiments, a drive spring (not shown) is under compression before device 10 is activated. A proximal end of the drive spring can be fixed within proximal region 21 of housing 11, and a distal end of the drive spring can be configured to apply a compressive force to a proximal surface of piston 23. Following activation, at least part of the energy stored in the drive spring can be applied to the proximal surface of piston 23. This compressive force can act on piston 23 to move it in a distal direction. Such distal movement acts to compress the liquid medicament within the syringe, forcing it out of needle 17.

Following injection, needle 17 can be retracted within sleeve 13 or housing 11. Retraction can occur when sleeve 13 moves distally as a user removes device 10 from a patient's body. This can occur as needle 17 remains fixedly located relative to housing 11. Once a distal end of sleeve 13 has moved past a distal end of needle 17, and needle 17 is covered, sleeve 13 can be locked. Such locking can include locking any proximal movement of sleeve 13 relative to housing 11.

Another form of needle retraction can occur if needle 17 is moved relative to housing 11. Such movement can occur if the syringe within housing 11 is moved in a proximal direction relative to housing 11. This proximal movement can be achieved by using a retraction spring (not shown), located in distal region 20. A compressed retraction spring, when activated, can supply sufficient force to the syringe to move it in a proximal direction. Following sufficient retraction, any relative movement between needle 17 and housing 11 can be locked with a locking mechanism. In addition, button 22 or other components of device 10 can be locked as required.

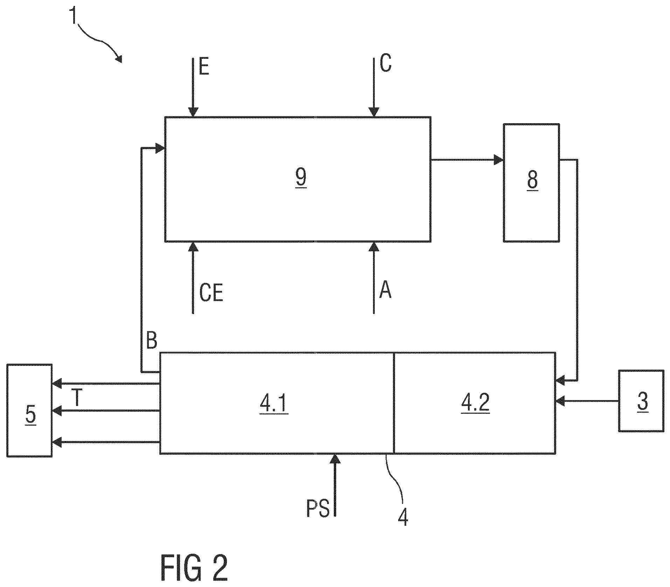

Figure 2 is a schematic view of a control loop 1 comprising a human body model 9 of a patient representing a controlled system. The human body has a defined consumption of glucose depending on physical activities A such as sleep with resting consumption and activity events with increased consumption. The glucose consumption furthermore depends on age and sex of the patient and on external conditions E such as the season (summer, winter etc.) or time of day. Blood glucose consumption is also a function of time. Sleep will typically lead to a decrease in blood glucose consumption. Carbohydrate exchange CE and physical activities A also influence blood glucose consumption and will cause a respective pulse response in blood glucose level. Furthermore, blood glucose consumption is influenced by characteristics C of diabetes such as type and degree of diabetes. Patients with type I diabetes do not produce insulin at all so only insulin delivered to the patient may be taken into account when calculating boluses. Patients with type II diabetes do produce different amounts of insulin depending on their degree of diabetes and exhibit various degrees of reuptake inhibition depending on weight and metabolic status which changes over the course of the year depending on the season.

The human body reacts to physical activities A and carbohydrate exchange CE like a PI (proportional integral) controller with a significant I (integral) component.

External conditions E and physical activities A can be determined automatically, e. g. by sensors and activity trackers. Characteristics C of diabetes and data relating carbohydrate exchange CE can be input into the human body model 9 by the user through a user interface. Likewise, the human body model 9 can estimate carbohydrate exchange based on previous inputs, in particular if the patient takes his meals regularly.

A glucose sensor 3 is applied to perform a blood glucose measurement, e.g. of venous blood of the human body. In an alternative embodiment, a blood glucose measurement of capillary blood may be performed by the glucose sensor 3, e.g. at the earlobe or on an eye by a glucose sensor 3 configured as an contact lens, wherein the pulse response is delayed as opposed to measurement of venous blood. The delay has to be taken into account as a dead time represented by a dead time element 8 when calculating a bolus B of insulin. The blood glucose measurement may be performed continuously, periodically, randomly or pseudo-randomly. The result of the blood glucose measurement is fed into a controller 4 comprising a bolus calculator 4.1. The controller 4 may furthermore comprise a comparator 4.2 for comparing blood glucose measurement values and blood glucose values calculated by the human body model 9 before handing over to the bolus calculator 4.1.

The bolus calculator 4.1 may be configured to calculate a bolus B for one type T of insulin or for selecting one out of a plurality of types T of insulin and calculate the bolus B for this selected type T.

Different types T of insulin have different active profiles, e.g. slowly acting insulin, rapid-acting insulin, medium acting insulin. The active profile of each type T of insulin is a pulse response to the administration of a bolus B of this type T of insulin. The pulse response depends on the pharmacokinetics of the selected type T of insulin, the amount of insulin in a bolus B and patient specifics PS such as age, sex, weight, body fat percentage. Elderly patients may have a slower metabolism than younger ones. Heavier patients may also need more insulin.

The patent specifics may be input into the bolus calculator 4.1 through a user interface and stored in the bolus calculator 4.1 such that they do not necessarily have to be input repeatedly.

The bolus calculator 4.1 convolutes a time series of blood glucose values which represent the pulse response of the human body with the known pulse response of one or more types T of insulin.

The convolution integral is determined by the equation

$$y(t) = \int_{-\infty}^{\infty} x(\tau)h(t-\tau)d\tau,$$

wherein $x(\tau)$ is a function exciting a system and $h(\tau)$ is the convolution function.

In the present embodiment, $h(t-\tau)$ is represented by the time series of measured blood glucose values of the human body over time and $x(\tau)$ is represented by the known pulse response (active profile) of one or more types T of insulin.

A characteristic map may be provided in the bolus calculator 4.1 comprising stored pulse responses for one or more types T of insulin for different sets of patent specifics such as age, sex, weight, body fat percentage.

The bolus calculator 4.1 may convolute all selectable types T of insulin for the set patient specifics with the time series of measured blood glucose values and select the most appropriate type T of insulin. This way, the most appropriate type T and bolus B of insulin may be selected.

The bolus B calculated by the bolus calculator 4.1 may then be delivered to the human body by a drug delivery device 5, e.g. an insulin pump or an insulin pen.

In an exemplary embodiment, the bolus calculator 4.1 may comprise a display for outputting the calculated bolus B and optionally the type T of insulin selected and a user may perform an injection according to the output using a drug delivery device 5 such as an insulin pen. The bolus may be manually set by the patient or automatically transmitted to the drug delivery device 5, e.g. by a wireless or wired connection, and automatically set by the drug delivery device 5. In another exemplary embodiment, the bolus calculator 4.1 may be connected to a drug delivery device 5 such as an insulin pump and control delivery of the calculated bolus B and optionally the type T of insulin selected.

Delivery of the bolus B will then cause a respective pulse response in the human body. Thus, the glucose level may be controlled to be within defined limits.

The types T of insulin which the bolus calculator 4.1 may select can comprise: a slowly acting insulin, a rapid-acting insulin, a medium acting insulin. In other embodiments, there may be only one, two or more than three types T of insulin which may be selected. For example, a slowly acting insulin may be used at nighttime.

A teach-in run may be performed with the controller 4 to measure the pulse response of the human body in order to determine the dead time to be taken into account in an actual application.

The bolus calculator 4.1 may also store measured blood glucose values and boluses B calculated to generate a history which may be taken into account when calculating future boluses B.

In the described system, blood glucose measurement values may be acquired by a continuous glucose measurement patch and may be transmitted to a mobile device, in particular in a wireless way, e.g. by NFC or Bluetooth® communication. Calory intake may be input manually or estimated by an application running on the mobile device. A health tracker may determine the heart rate and transmit it, in particular in a wireless way, e.g. by NFC or Bluetooth® communication. Injection of insulin may be performed through the abdominal wall into the fat tissue by a pen injector or an insulin pump.

The body model 9 may be a time-discrete system/control circuit which may typically be operated in the frequency domain. All values are present as discrete values in the time domain and may be processed as such. The control circuit may be described in the time domain but may first have to be subjected to a Z transformation.

The comparator 4.2 may be set to glucose target values, in particular an upper limit value and a lower limit value, e.g. an upper limit value of 150 mg/dL and a lower limit value of 80mg/dL. After running simulations with changing insulin values, the bolus calculator 4.1 determines an optimum and outputs it.

The complex controlled system body model 9 may be a system of differential equations which is numerically solved and outputs the course of the blood glucose level (BGM) over time which, after having passed the dead time element 8, is compared with the measured values of the glucose sensor 3 (continuous glucose measurement) over time.

The course of the blood glucose level BGM may be calculated by BGM(t- to) = F(t, $\tau$, f(carb, HF, ..)) with carb being the carbohydrate exchange CE and HF being the heart rate.

As the system is sampled, it may be transformed into the frequency domain by a Z transformation and back into the time domain.

The bolus calculator 4.1 can convolute all selectable types of insulin T for the set patient characteristics with the time series of the measured blood glucose values and select the most appropriate type T of insulin and determine the appropriate bolus B.

This may be achieved by repeated simulation towards the glucose target range. The patient typically decides when to perform the injections (typically after the meals). The optimization of the bolus B may then be performed at that point in time, either outputting a value which is to be confirmed or by controlling a pump which then may deliver the bolus.

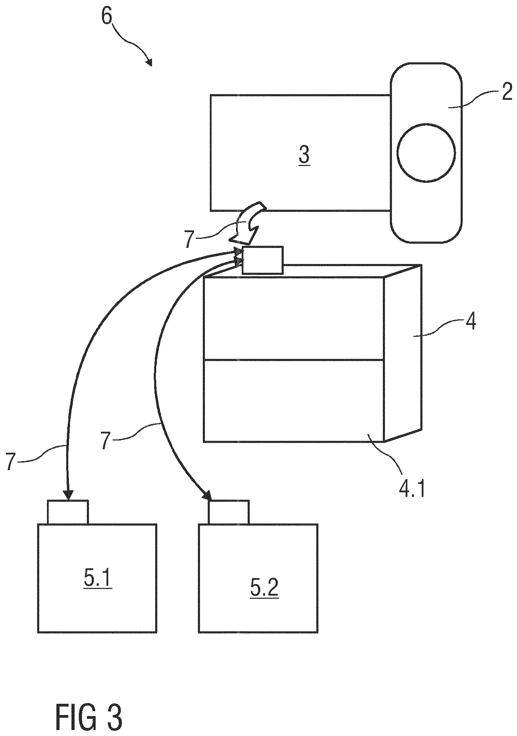

Figure 3 is a schematic view of an exemplary embodiment of an arrangement 6 for determining a bolus B of insulin. A blood glucose sensor 3 is attached to or implanted in a human body 2 and adapted to determine blood glucose values, e.g. by capillary glucose measurement. A controller 4, e.g. a mobile device such as a smart phone, is arranged to communicate with the blood glucose sensor 3 by a wired or wireless connection 7, e.g. Bluetooth®. The controller 4 comprises a bolus calculator 4.1, e.g. a software application. The controller 4 may also be connected to one or more drug delivery devices 5, e.g. an insulin pen 5.1 and an insulin pump 5.2 by a wired or wireless connection 7, e.g. Bluetooth®.

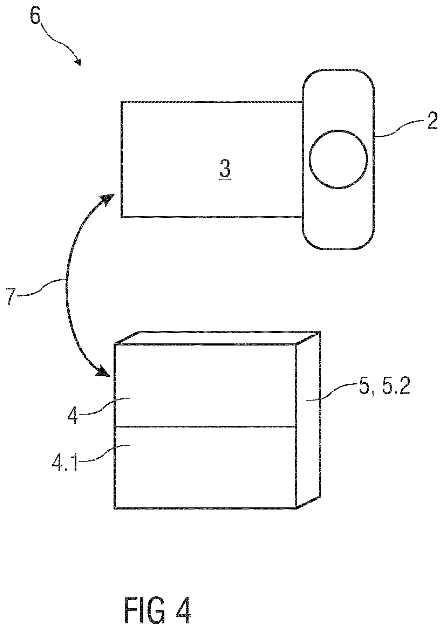

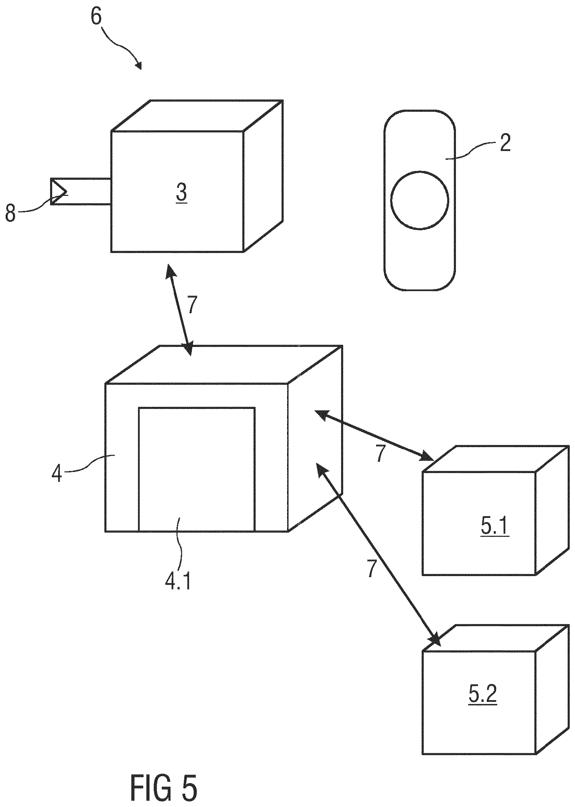

Figure 4 is a schematic view of an exemplary embodiment of an arrangement 6 for determining a bolus B of insulin. A blood glucose sensor 3 is attached to or implanted in a human body 2 and adapted to determine blood glucose values, e.g. by capillary glucose measurement. A controller 4 is integrated with a drug delivery device 5, e.g. an insulin pump 5.2, and arranged to communicate with the blood glucose sensor 3 by a wired or wireless connection 7, e.g. Bluetooth®. The controller 4 comprises a bolus calculator 4.1, e.g. a software application. Figure 5 is a schematic view of an exemplary embodiment of an arrangement 6 for determining a bolus B of insulin. A blood glucose sensor 3 is arranged to analyse blood glucose measurement strips 8 with blood samples from a human body 2 and adapted to determine blood glucose values. A controller 4, e.g. a mobile device such as a smart phone, is arranged to communicate with the blood glucose sensor 3 by a wired or wireless connection 7, e.g. Bluetooth®. The controller 4 comprises a bolus calculator 4.1, e.g. a software application. The controller 4 may also be connected to one or more drug delivery devices 5, e.g. two or more different insulin pens 5.1, 5.3, one with a slowly acting insulin, e.g. Lantus® and another one with a rapid-acting insulin, e.g. Apidra®.

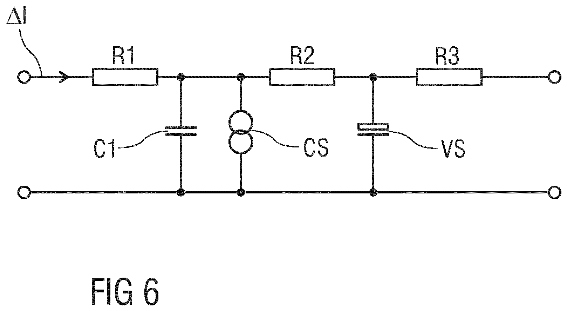

Figure 6 is a schematic view of a simplified body model depicted as an electronic circuit in the form of a quadrupole comprising a low pass circuit formed by a first resistor R1 and a capacitor C1 and a controlled current source CS in parallel to the capacitor C1, the low pass circuit followed by a second resistor R2, a voltage source VS, e.g. a battery, and a third R3.

An insulin injection ΔI affects the model like a pulse affects a quadrupole, i.e. proportional to a decrease in the blood glucose level -ΔBG. The low pass circuit represents the attenuation by the abdominal wall and its fat tissue until the insulin is distributed through the venous system of the organism. The controlled current source CS represents calory consumption by activity and calory intake, i.e. increase or decrease of the blood glucose level. The central fat tissue and the other tissue, in particular the liver is represented by the voltage source VS. Even if no food intake occurs, the voltage source VS feeds glucose into the system from the fat tissue (controlled by the insulin intake ΔI depending on the degree of diabetes).

The terms "drug" or "medicament" are used herein to describe one or more pharmaceutically active compounds. As described below, a drug or medicament can include at least one small or large molecule, or combinations thereof, in various types of formulations, for the treatment of one or more diseases. Exemplary pharmaceutically active compounds may include small molecules; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more of these drugs are also contemplated.

The term "drug delivery device" shall encompass any type of device or system configured to dispense a drug into a human or animal body. Without limitation, a drug delivery device may be an injection device (e.g., syringe, pen injector, auto injector, large-volume device, pump, perfusion system, or other device configured for intraocular, subcutaneous, intramuscular, or intravascular delivery), skin patch (e.g., osmotic, chemical, microneedle), inhaler (e.g., nasal or pulmonary), implantable (e.g., coated stent, capsule), or feeding systems for the gastrointestinal tract. The presently described drugs may be particularly useful with injection devices that include a needle, e.g., a small gauge needle.

The drug or medicament may be contained in a primary package or "drug container" adapted for use with a drug delivery device. The drug container may be, e.g., a cartridge, syringe, reservoir, or other vessel configured to provide a suitable chamber for storage (e.g., short- or long-term storage) of one or more pharmaceutically active compounds. For example, in some instances, the chamber may be designed to store a drug for at least one day (e.g., 1 to at least 30 days).

In some instances, the chamber may be designed to store a drug for about 1 month to about 2 years. Storage may occur at room temperature (e.g., about 20°C), or refrigerated temperatures (e.g., from about -4°C to about 4°C). In some instances, the drug container may be or may include a dual-chamber cartridge configured to store two or more components of a drug formulation (e.g., a drug and a diluent, or two different types of drugs) separately, one in each chamber. In such instances, the two chambers of the dual-chamber cartridge may be configured to allow mixing between the two or more components of the drug or medicament prior to and/or during dispensing into the human or animal body. For example, the two chambers may be configured such that they are in fluid communication with each other (e.g.,

by way of a conduit between the two chambers) and allow mixing of the two components when desired by a user prior to dispensing. Alternatively or in addition, the two chambers may be configured to allow mixing as the components are being dispensed into the human or animal body.

The drug delivery devices and drugs described herein can be used for the treatment and/or prophylaxis of many different types of disorders. Exemplary disorders include, e.g., diabetes mellitus or complications associated with diabetes mellitus such as diabetic retinopathy, thromboembolism disorders such as deep vein or pulmonary thromboembolism. Further exemplary disorders are acute coronary syndrome (ACS), angina, myocardial infarction, cancer, macular degeneration, inflammation, hay fever, atherosclerosis and/or rheumatoid arthritis.

Exemplary drugs for the treatment and/or prophylaxis of diabetes mellitus or complications associated with diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the term "derivative" refers to any substance which is sufficiently structurally similar to the original substance so as to have substantially similar functionality or activity (e.g., therapeutic effectiveness).

Exemplary insulin analogues are Gly(A21), Arg(B31), Arg(B32) human insulin (insulin glargine); Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Exemplary insulin derivatives are, for example, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28-ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin; B29-N-(N-lithocholyl-gamma-glutamyl)-des(B30) human insulin; B29-N-(ω-carboxyheptadecanoyl)-des(B30) human insulin and B29-N-(ω-carboxyheptadecanoyl) human insulin. Exemplary GLP-1, GLP-1 analogues and GLP-1 receptor agonists are, for example: Lixisenatide / AVE0010 / ZP10 / Lyxumia, Exenatide / Exendin-4 / Byetta / Bydureon / ITCA 650 / AC-2993 (a 39 amino acid peptide which is produced by the salivary glands of the Gila monster), Liraglutide / Victoza, Semaglutide, Taspoglutide, Syncria / Albiglutide, Dulaglutide, rExendin-4, CJC-1134-PC, PB-1023, TTP-054, Langlenatide / HM-11260C, CM-3, GLP-1 Eligen, ORMD-0901, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, TT-401, BHM-034. MOD-6030, CAM-2036, DA-15864, ARI-2651, ARI-2255, Exenatide-XTEN and Glucagon-Xten.

An exemplary oligonucleotide is, for example: mipomersen / Kynamro, a cholesterol-reducing antisense therapeutic for the treatment of familial hypercholesterolemia.

Exemplary DPP4 inhibitors are Vildagliptin, Sitagliptin, Denagliptin, Saxagliptin, Berberine.

Exemplary hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.

Exemplary polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 / Synvisc, a sodium hyaluronate.

The term "antibody", as used herein, refers to an immunoglobulin molecule or an antigen-binding portion thereof. Examples of antigen-binding portions of immunoglobulin molecules include F(ab) and F(ab')$_2$ fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region.

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present disclosure include, for example, Fab fragments, F(ab')2 fragments, scFv (single-chain Fv) fragments, linear antibodies, monospecific or multispecific antibody fragments such as bispecific, trispecific, and multispecific antibodies (e.g., diabodies, triabodies, tetrabodies), minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen.

Exemplary antibodies are anti PCSK-9 mAb (e.g., Alirocumab), anti IL-6 mAb (e.g., Sarilumab), and anti IL-4 mAb (e.g., Dupilumab).

The compounds described herein may be used in pharmaceutical formulations comprising (a) the compound(s) or pharmaceutically acceptable salts thereof, and (b) a pharmaceutically acceptable carrier. The compounds may also be used in pharmaceutical formulations that include one or more other active pharmaceutical ingredients or in pharmaceutical formulations in which the present compound or a pharmaceutically acceptable salt thereof is the only active ingredient. Accordingly, the pharmaceutical formulations of the present disclosure encompass any formulation made by admixing a compound described herein and a pharmaceutically acceptable carrier.

Pharmaceutically acceptable salts of any drug described herein are also contemplated for use in drug delivery devices. Pharmaceutically acceptable salts are for example acid addition salts and basic salts. Acid addition salts are e.g. HCl or HBr salts. Basic salts are e.g. salts having a cation selected from an alkali or alkaline earth metal, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are known to those of skill in the arts.

Pharmaceutically acceptable solvates are for example hydrates or alkanolates such as methanolates or ethanolates.

List of References

| 1 | control loop |
|---|---|
| 2 | human body |
| 3 | glucose sensor |
| 4 | controller |
| 4.1 | bolus calculator |
| 4.2 | comparator |
| 5 | drug delivery device |
| 5.1 | insulin pen |
| 5.2 | insulin pump |
| 5.3 | insulin pen |
| 6 | arrangement |
| 7 | connection |
| 8 | dead time element |
| 9 | human body model |
| 10 | drug delivery device |
| 11 | housing |
| 12 | cap assembly |
| 13 | needle sleeve |
| 17 | needle |
| 20 | distal region |
| 21 | proximal region |
| 22 | button |
| 23 | piston |
| A | physical activities |
| B | bolus |
| C | characteristics of diabetes |
| CE | carbohydrate exchange |
| E | external condiditons |
| T | type of insulin |
| X | longitudinal axis |
| PS | patient specifics |

**Claims**

1. A bolus calculator (4.1) for determining a bolus (B) of insulin, the bolus calculator (4.1) having an input configured to be fed with a time series $(h(t-\tau))$ of measured blood glucose values of a human body and to store at least one known pulse response $(x(\tau))$ representing an active profile of at least one insulin, wherein the bolus calculator (4.1) is configured to convolute the time series $(h(t-\tau))$ of the blood glucose values with the known pulse response $(x(\tau))$ to obtain the bolus (B) **characterized in that** the bolus calculator (4.1) is configured to select one out of a plurality of types (T) of insulin and to calculate the bolus (B) for this selected type (T).

2. The bolus calculator (4.1) of claim 1, further configured to take into account a dead time in the time series $(h(t-\tau))$ due to blood glucose measurement of capillary blood of a human body (2).

3. The bolus calculator (4.1) according to any one of the preceding claims, further configured to store and take into account patient specifics (PS), in particular at least one of age, sex, weight and body fat percentage.

4. The bolus calculator (4.1) according to claim 3, further configured to store a characteristic map comprising a plurality of stored pulse responses (x($\tau$)) for one or more types (T) of insulin for different sets of patent specifics (PS).

5. An arrangement (6) for determining a bolus (B) of insulin, comprising the bolus calculator (4.1) according to any one of the preceding claims, a human body model (9), a blood glucose sensor (3) adapted to perform a blood glucose measurement of venous blood or capillary blood of a human body (2) and a comparator (4.2) for comparing blood glucose values measured by the blood glucose sensor (3) with blood glucose values calculated by the human body model (9) before handing over to the bolus calculator (4.1).

6. A method for calculating a bolus (B) of insulin, comprising feeding a time series (h(t-$\tau$)) of measured blood glucose values of a human body into an input of a bolus calculator (4.1) and storing at least one known pulse response (x($\tau$)) representing an active profile of at least one insulin in the bolus calculator (4.1), convoluting the time series (h(t-$\tau$)) of the blood glucose values with the known pulse response (x($\tau$)) to obtain the bolus (B), **characterized in that** one out of a plurality of types (T) of insulin is selected and the bolus (B) for this selected type (T) is calculated.

7. The method of claim 6, wherein a dead time in the time series (h(t-$\tau$)) due to blood glucose measurement of capillary blood of a human body (2) is taken into account.

8. The method of any one of claims 6 or 7, comprising storing and taking into account patient specifics (PS), in particular at least one of age, sex, weight and body fat percentage.

9. The method according to claim 8, further comprising storing a characteristic map comprising a plurality of stored pulse responses (x($\tau$)) for one or more types (T) of insulin for different sets of patient specifics (PS).

10. The method according to claim 8 or 9, further comprising convoluting all selectable types (T) of insulin for the set patient specifics (PS) with the time series (h(t-$\tau$)) of measured blood glucose values and selecting the most appropriate type (T) of insulin.

11. The method according to any one of claims 6 to 10, further comprising performing a teach-in run to measure a pulse response of the human body (2) in order to determine the dead time.

12. The method according to any one of the claims 6 to 11, further comprising storing measured blood glucose values and boluses (B) calculated to generate a history and taking the history into account when calculating boluses (B).

13. The method according to any one of the claims 6 to 12, further comprising comparing blood glucose values measured by a blood glucose sensor (3) with blood glucose values calculated by a human body model (9) before handing over to the bolus calculator (4.1).

**Patentansprüche**

1. Bolusrechner (4.1) zum Bestimmen eines Bolus (B) von Insulin, wobei der Bolusrechner (4.1) einen Eingang aufweist, der so konfiguriert ist, dass er mit einer Zeitreihe (h(t-$\tau$)) von gemessenen Blutglukosewerten eines menschlichen Körpers gespeist wird und mindestens eine bekannte Pulsantwort (x($\tau$)) speichert, die ein aktives Profil von mindestens einem Insulin darstellt, wobei der Bolusrechner (4.1) dazu konfiguriert ist, die Zeitreihe (h(t-$\tau$)) der Blutglukosewerte mit der bekannten Pulsantwort (x($\tau$)) zu falten, um den Bolus (B) zu erhalten, **dadurch gekennzeichnet, dass** der Bolusrechner (4.1) dazu konfiguriert ist, einen aus mehreren Typen (T) von Insulin auszuwählen und den Bolus (B) für diesen ausgewählten Typ (T) zu berechnen.

2. Bolusrechner (4.1) nach Anspruch 1, der ferner dazu konfiguriert ist, eine Totzeit in der Zeitreihe (h(t-$\tau$)) aufgrund einer Blutglukosemessung von Kapillarblut eines menschlichen Körpers (2) zu berücksichtigen.

3. Bolusrechner (4.1) nach einem der vorhergehenden Ansprüche, der ferner dazu konfiguriert ist, Patientenbesonderheiten (PS), insbesondere mindestens eines von Alter, Geschlecht, Gewicht und Körperfettprozentsatz, zu speichern und zu berücksichtigen.

4. Bolusrechner (4.1) nach Anspruch 3, der ferner dazu konfiguriert ist, eine charakteristische Abbildung mit mehreren gespeicherten Pulsantworten (x($\tau$)) für einen oder mehrere Typen (T) von Insulin für verschiedene Sätze von Patientenbesonderheiten (PS) zu speichern.

5. Anordnung (6) zum Bestimmen eines Bolus (B) von Insulin, die den Bolusrechner (4.1) nach einem der vorhergehenden Ansprüche, ein Modell (9) eines menschlichen Körpers, einen Blutglukosesensor (3), der dazu ausgelegt ist, eine Blutglukosemessung von Venenblut oder Kapillarblut eines menschlichen Körpers (2) durchzuführen, und einen Komparator (4.2) zum Vergleichen von durch den Blutglukosesensor (3) gemessenen Blutglukosewerten mit durch das Modell (9) eines menschlichen Körpers berechneten Blutglukosewerten vor der Übergabe an den Bolusrechner (4.1) umfasst.

6. Verfahren zum Berechnen eines Bolus (B) von Insulin, das das Einspeisen einer Zeitreihe (h(t-τ)) von gemessenen Blutglukosewerten eines menschlichen Körpers in einen Eingang eines Bolusrechners (4.1) und das Speichern mindestens einer bekannten Pulsantwort (x(τ)), die ein aktives Profil von mindestens einem Insulin darstellt, im Bolusrechner (4.1), das Falten der Zeitreihe (h(t-τ)) der Blutglukosewerte mit der bekannten Pulsantwort (x(τ)), um den Bolus (B) zu erhalten, umfasst, **dadurch gekennzeichnet, dass** einer aus mehreren Typen (T) von Insulin ausgewählt wird und der Bolus (B) für diesen ausgewählten Typ (T) berechnet wird.

7. Verfahren nach Anspruch 6, wobei eine Totzeit in der Zeitreihe (h(t-τ)) aufgrund einer Blutglukosemessung von Kapillarblut eines menschlichen Körpers (2) berücksichtigt wird.

8. Verfahren nach einem der Ansprüche 6 oder 7, das das Speichern und Berücksichtigen von Patientenbesonderheiten (PS), insbesondere mindestens eines von Alter, Geschlecht, Gewicht und Körperfettprozentsatz, umfasst.

9. Verfahren nach Anspruch 8, das ferner das Speichern einer charakteristischen Abbildung mit mehreren gespeicherten Pulsantworten (x(τ)) für einen oder mehrere Typen (T) von Insulin für verschiedene Sätze von Patientenbesonderheiten (PS) umfasst.

10. Verfahren nach Anspruch 8 oder 9, das ferner das Falten aller auswählbaren Typen (T) von Insulin für die festgelegten Patientenbesonderheiten (PS) mit der Zeitreihe (h(t-τ)) von gemessenen Blutglukosewerten und das Auswählen des geeignetsten Typs (T) von Insulin umfasst.

11. Verfahren nach einem der Ansprüche 6 bis 10, das ferner das Durchführen eines Anlernlaufs umfasst, um eine Pulsantwort des menschlichen Körpers (2) zu messen, um die Totzeit zu bestimmen.

12. Verfahren nach einem der Ansprüche 6 bis 11, das ferner das Speichern von gemessenen Blutglukosewerten und berechneten Boli (B), um einen Verlauf zu erzeugen, und das Berücksichtigen des Verlaufs, wenn Boli (B) berechnet werden, umfasst.

13. Verfahren nach einem der Ansprüche 6 bis 12, das ferner das Vergleichen von durch einen Blutglukosesensor (3) gemessenen Blutglukosewerten mit durch ein Modell (9) eines menschlichen Körpers berechneten Blutglukosewerten vor der Übergabe an den Bolusrechner (4.1) umfasst.

**Revendications**

1. Calculateur de bolus (4.1) destiné à déterminer un bolus (B) d'insuline, le calculateur de bolus (4.1) étant pourvu d'une entrée configurée pour recevoir une série temporelle (h(t-T)) de valeurs de glycémie mesurées d'un corps humain et pour stocker au moins une réponse impulsionnelle (x(τ)) connue représentant un profil actif d'au moins une insuline, le calculateur de bolus (4.1) étant configuré pour effectuer une convolution de la série temporelle (h(t-T)) des valeurs de glycémie avec la réponse impulsionnelle (x(τ)) connue afin d'obtenir le bolus (B), le calculateur de bolus (4.1) étant **caractérisé en ce qu'**il est configuré pour sélectionner l'un d'une pluralité de types (T) d'insuline et pour calculer le bolus (B) pour ce type (T) sélectionné.

2. Calculateur de bolus (4.1) selon la revendication 1, configuré en outre pour prendre en compte un temps mort dans la série temporelle (h(t-T)) dû à une mesure de glycémie sur du sang capillaire d'un corps humain (2).

3. Calculateur de bolus (4.1) selon l'une quelconque des revendications précédentes, configuré en outre pour stocker et prendre en compte des particularités du patient (PS), notamment au moins un élément parmi l'âge, le sexe, le poids et le pourcentage de graisse corporelle.

4. Calculateur de bolus (4.1) selon la revendication 3, configuré en outre pour stocker une table de caractéristiques comprenant une pluralité de réponses impulsionnelles (x(τ)) stockées pour un ou plusieurs types (T) d'insuline pour différents ensembles de particularités du patient (PS).

5. Agencement (6) destiné à déterminer un bolus (B) d'insuline, comprenant le calculateur de bolus (4.1) selon l'une quelconque des revendications précédentes, un modèle de corps humain (9), un capteur de glycémie (3) adapté à réaliser une mesure de glycémie sur du sang veineux ou du sang capillaire d'un corps humain (2) et un comparateur (4.2) destiné à comparer des valeurs de glycémie mesurées par le capteur de glycémie (3) avec des valeurs de glycémie calculées par le modèle de corps humain (9) avant la prise en charge par le calculateur de bolus (4.1).

**6.** Procédé de calcul d'un bolus (B) d'insuline, comprenant l'introduction d'une série temporelle (h(t-τ)) de valeurs de glycémie mesurées d'un corps humain à une entrée d'un calculateur de bolus (4.1) et le stockage, dans le calculateur de bolus (4.1), d'au moins une réponse impulsionnelle (x(τ)) connue représentant un profil actif d'au moins une insuline, la convolution de la série temporelle (h(t-T)) des valeurs de glycémie avec la réponse impulsionnelle (x(τ)) connue afin d'obtenir le bolus (B), **caractérisé par** la sélection de l'un d'une pluralité de types (T) d'insuline et le calcul du bolus (B) pour ce type (T) sélectionné.

**7.** Procédé selon la revendication 6, dans lequel un temps mort dans la série temporelle (h(t-τ)) dû à une mesure de glycémie sur du sang capillaire d'un corps humain (2) est pris en compte.

**8.** Procédé selon l'une quelconque des revendications 6 ou 7, comprenant le stockage et la prise en compte de particularités du patient (PS), notamment d'au moins un élément parmi l'âge, le sexe, le poids et le pourcentage de graisse corporelle.

**9.** Procédé selon la revendication 8, comprenant en outre le stockage d'une table de caractéristiques comprenant une pluralité de réponses impulsionnelles (x(τ)) stockées pour un ou plusieurs types (T) d'insuline pour différents ensembles de particularités du patient (PS).

**10.** Procédé selon la revendication 8 ou 9, comprenant en outre la convolution de tous les types (T) sélectionnables d'insuline pour les particularités du patient (PS) définies avec la série temporelle (h(t-T)) de valeurs de glycémie mesurées et la sélection du type (T) d'insuline le plus approprié.

**11.** Procédé selon l'une quelconque des revendications 6 à 10, comprenant en outre la réalisation d'un cycle d'apprentissage afin de mesurer une réponse impulsionnelle du corps humain (2) en vue de déterminer le temps mort.

**12.** Procédé selon l'une quelconque des revendications 6 à 11, comprenant en outre le stockage de valeurs de glycémie mesurées et de bolus (B) calculés afin de générer un historique et la prise en compte de l'historique lors du calcul de bolus (B).

**13.** Procédé selon l'une quelconque des revendications 6 à 12, comprenant en outre la comparaison de valeurs de glycémie mesurées par un capteur de glycémie (3) avec des valeurs de glycémie calculées par un modèle de corps humain (9) avant la prise en charge par le calculateur de bolus (4.1).

FIG 1A
Prior Art

FIG 1B
Prior Art

FIG 2

FIG 3

FIG 4

FIG 5

FIG 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2013184896 A1

- US 2009006129 A1